# EUROPEAN PATENT SPECIFICATION

(11) **EP 2 080 512 B1**
(45) Date of publication and mention of the grant of the patent: **07.03.2012**
(21) Application number: 07829485.7
(22) Date of filing: 10.10.2007
(51) Int. Cl.: A61K 31/357, A61P 35/00, C07D 323/00

(54) **Anti-cancer agent**
Mittel gegen Krebs
Agent anti-cancer

(30) Priority: 14.10.2006 JP 2006280802
(43) Date of publication of application: 22.07.2009
(73) Proprietor: Wan Station Co., Ltd, Tokyo 111-0033 (JP)
(72) Inventor: ONO, Nobufumi, Fukuoka-shi Fukuoka 814-0153 (JP)
(74) Representative: Duckworth, Timothy John
(86) International application number: PCT/JP2007/069747
(87) International publication number: WO 2008/044704

(56) References cited:
- EP-A1- 1 724 265
- JP-A- 2005 247 790
- KIHARA N. ET AL.: 'Direct preparation of rotaxane from aminoalcohol: selective O-acylation of aminoalcohol in the presence of trifluoromethanesulfonic acid and crown ether' CHEMISTRY LETTERS vol. 30, no. 6, 2001, pages 592 - 593, XP003022166
- TEODORI E. ET AL.: 'Exploratory Chemistry toward the Identification of a New Class of Multidrug REsistance Reverters Inspired by Pervilleine and Verapamil Models' JOURNAL OF MEDICINAL CHEMISTRY vol. 48, no. 23, 2005, pages 7426 - 7436, XP003022167

## Description

### Technical Field

The present invention relates to an anti-cancer agent, and particularly to an anti-cancer agent comprising a rotaxane compound as an active ingredient.

### Background Art

In Japan, the average life expectancy was extended by 10 years or more, when compared with 30 years ago. The major cause is considered to be the development of a large number of therapeutic agents for apoplectic stroke or heart diseases, which have often become a cause of death in past times. However, as the number of such apoplectic stroke or heart diseases serving as a cause of death has decreased, the number of deaths by cancer (malignant neoplasm) has increased year by year. At present, the first place for a cause of death is cancer, and thus a large number of researchers are currently developing anti-cancer agents.

A majority of anti-cancer agents exhibit their action after they are absorbed into cancer cells. Hence, it has conventionally been considered that an agent with a small molecular weight, which has a stable structure, is effective for easy incorporation of the agent into cells via a receptor.
Patent Document 1: JP 3741706 B.
Non-Patent Document 1: Toshikazu TAKATA, Nobuhiro KIHARA & Yoshio FURUSHO: "Functionality design of nano materials by synthesis of interlocked compounds" Kobunshi, 2001, 50 (11), p770-773.
Non-Patent Document 2: Yoshio FURUSHO, Nobuhiro KIHARA & Toshikazu TAKATA: "Synthesis of rotaxane and polyrotaxane exploiting slipping reaction: New synthetic method of polymeric materials" Polym. Appl. 2001, 50(3), p114-120. Non-Patent Document 3: Nobuhiro KIHARA & Toshikazu TAKATA: "Efficient syntheses of interlocked molecules based on hydrogen-bonding: Recent progress in syntheses of rotaxanes and catenanes" Yuki Gosei Kagaku Kyokaishi, 2001, 59(3), p206-218.

Further Non-Patent Document: Nobushiro Kihara, Jong-II Shin, Yasuko Ohga, and Toshikazu Takata: "Direct Preparation of Rotaxane from Aminoalcohol: Selectictive O-Acylation of Aminoalcohol in the Presence of Trifluoromethanesufonic Acid and Crown Ether" Chemistry Letters, 2001, 30(6), p592-593. This document discloses the preparation of the compound of Formula (I) as depicted below with trifluoromethanesulfonic acid as catalyst. No pharmacological activity is described.

### Disclosure of the Invention

Nevertheless, such an anti-cancer agent that is readily absorbed into cells via a receptor is often readily excreted from the cells. Thus, the anti-cancer agent has been problematic in that it is less likely to remain in cancer cells. Moreover, cancer cells that once excrete an anti-cancer agent may become resistant to the anti-cancer agent. Hence, the same anti-cancer agent as once applied becomes ineffective on the cancer cells.

Moreover, the anti-cancer agent that is readily absorbed into cancer cells via a receptor is also readily introduced into normal cells. Thus, the anti-cancer agent has also been problematic in that it is difficult for the agent to be used in local treatment and in that side effects are likely to occur with the agent.

In order to solve the aforementioned problems, the present inventor had conducted intensive studies directed towards developing a novel anti-cancer agent that is less likely to be excreted from cancer cells. As a result, the inventor has succeeded in developing an anti-cancer agent comprising a catenane compound as an active ingredient (see Patent Document 1). The catenane having anti-cancer action is a type of compound called amide-type [2] catenane, and it has a structure in which two molecular rings that are not covalently bound to each other are ligated to each other like a chain.

A novel polymer compound such as catenane whose part is not bound via a covalent bond has a relatively large molecular weight, and the molecule readily varies. Thus, it is considered that such compound is not readily excreted from cancer cells if it has once been introduced therein. Moreover, in general, such novel polymer compound is less likely to be absorbed into cells. Accordingly, if such compound is used for cancer cells locally, it would become possible to effectively suppress the proliferation of cancer cells, while reducing its influence upon normal cells to a minimum.

In addition to catenane, a compound called rotaxane has been known as another example of the novel polymer compound (see Non-Patent Documents 1-3). In Patent Document 1, the present inventor had predicted that such rotaxane would have an anti-cancer action. In order to demonstrate this prediction, the present inventor has conducted intensive studies.

The present invention has been completed under such circumstances. It is an object of the present invention to provide an anti-cancer agent comprising a rotaxane compound as an active ingredient.

The present inventor has conducted intensive studies directed towards discovering a rotaxane compound having anti-cancer action. As a result, the inventor has confirmed that a part of rotaxane compounds has anti-cancer action.

Specifically, the present invention provides a rotaxane compound represented by the following chemical formula (I) for use as an anti-cancer agent: 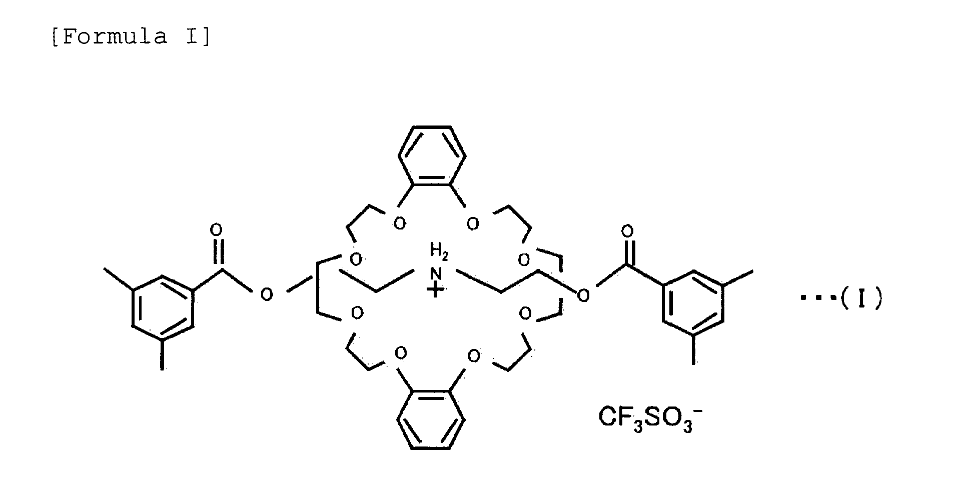 The invention further provides a composition for use as an anti-cancer agent which comprises a rotaxane compound of the invention dissolved in a solution, wherein the concentration of said rotaxane compound in said solution is 10 nM or more.

This rotaxane compound is formally called [2][bis (2-(3,5-dimethylphenylcarbonyloxy) ethyl) ammonium trifluoromethanesulfonate]-[dibenzo-24-crown-8] rotaxane. Its molecular weight is 819. The rotaxane compound is relatively heat-stable, and is organic-solvent-soluble and water-insoluble.

As shown in the aforementioned chemical formula (I), the rotaxane compound consists of two moieties; namely, a rod-like moiety, to both ends of which relatively large molecules have bound, and a cyclic moiety (a crown ether) into which the rod-like moiety has been inserted. Since the rod-like moiety has been inserted into the cyclic moiety, the cyclic moiety can move along the rod-like moiety as an axis. Since, however, relatively large molecules have bound to both ends of the rod-like moiety, the molecules serve as stoppers to prevent the cyclic moiety to detach from the rod-like moiety.

Hence, although the rod-like moiety is ligated to the cyclic moiety, the two moieties do not bind to each other via a covalent bond. Thus, the shape of the molecule varies readily, and the compound is relatively less likely to be introduced into cells via a receptor. If, however, the rotaxane compound is introduced into cancer cells, the proliferation of the cells is suppressed.

In order to introduce such rotaxane compound into cancer cells, it is preferable to create pores on the cell membrane of the cancer cells and introduce the compound into the cells through the pores. As a method of creating pores on the cell membrane, an electroporation method can be employed. If the electroporation method is employed, the rotaxane compound can be locally introduced, and the amount of the compound introduced into normal cells can be reduced to a minimum. As a result, it becomes possible to reduce a burden on patients, such as side effects.

At present, the rotaxane compound whose anti-cancer action has been confirmed by the present inventor is only the rotaxane compound represented by the above-described chemical formula. However, considering the general characteristics of rotaxane compounds that they are less likely to be introduced into cells and that they are not readily excreted therefrom if they are once introduced therein, it is assumed that some other rotaxane compounds also have anti-cancer action, as well as the rotaxane compound represented by the above described chemical formula.

Hence, according to the present invention, it becomes possible to provide an anti-cancer agent, which is less likely to be excreted from cancer cells and is preferable used in local treatment.

### Brief Description of the Drawings

[Fig.1] A graph showing the evaluation results of samples A0 to A5.
[Fig.2] A graph showing the evaluation results of samples B0 to B4.
[Fig.3] A graph showing the evaluation results of samples C0 to C2.
[Fig.4] A graph showing the evaluation results of samples D0 to D2.
[Fig.5] A table showing the results obtained by comparing the anti-cancer agent of the present invention with a conventional anti-cancer agent.

### Best Mode for Carrying Out the Invention

The preferred embodiments of the present invention will be described in detail below.

The anti-cancer agent according to the present invention comprises the rotaxane compound represented by the above-described chemical formula (I) as an active ingredient. A method of synthesizing the rotaxane compound is not particularly limited. It can be synthesized by a slipping reaction method.

Since the rotaxane compound thus obtained can be dissolved in a solvent such as dimethyl sulfoxide (DMSO), the obtained solution can be used as an anti-cancer agent. Though the concentration of rotaxane in the solution is not particularly limited, it is preferably 10 nM (nanomolar concentration), and more preferably 100 nM or more. This is because, though the rotaxane concentration less than 10 nM in the solution has an effect of retarding the proliferation of cancer cells to a certain extent, the concentration of 10 nM or more has a clear effect of retarding the cancer cell proliferation. Moreover, if the concentration of rotaxane is set at 100 nM or more, its effect of suppressing the proliferation of cancer cells becomes significant, and thus it becomes possible to reduce the cancer cells over time.

The "rod-like moiety" and the "cyclic moiety" that constitute the rotaxane compound do not bind to each other via a covalent bond. Thus, its shape varies readily, and it is relatively less likely to be introduced into cells via a receptor. Accordingly, in order to introduce such rotaxane compound into a cancer cell, it is preferable to create pores on the cell membrane of the cancer cell by employing an electroporation method. The pores formed on the cell membrane by the electroporation are rapidly repaired after application of electric voltage has been terminated. As to the voltage waveform applied during the electroporation, a DC pulse is preferable, and it is preferable apply the pulse 10 times, with a pulse voltage of 10 to 40 V, a pulse width of approximately 10 msec, and a pulse cycle of approximately 3 sec.

If such rotaxane compound is introduced into cancer cells by the electroporation method or the like, it acts in the cells for a long time because the cancer cells cannot readily excrete the compound. Moreover, resistance to the rotaxane compound is less likely to appear.

### [Examples]

Next, the examples of the present invention will be described.

Before examining the anti-cancer action of the rotaxane compound, the concentration of a solvent and conditions for electroporation were determined. With regard to the solvent concentration, since DMSO used as a solvent acts as a radical scavenger on cancer cells, it is necessary to examine the anti-cancer action of the rotaxane compound in a state where the action of DMSO as a radical scavenger can be substantially neglected. On the other hand, with regard to the electroporation conditions, since too high electric voltage applied during electroporation causes cytotoxicity, it is necessary to examine the anti-cancer action in a state where the influence of the electroporation on cancer cells can be substantially neglected.

### [Determination of solvent concentration]

First, a mouse malignant melanoma cell line (B16) was prepared, and approximately 5,000 cells were seeded into each well of a 96-well microplate. Thereafter, DMSO was added to each well to result in a total amount of 100 µL. As for samples A0 to A5, the concentration of DMSO added was set as shown in Table 1.

**[Table 1]**

| Sample name | DMSO concentration |
|---|---|
| Sample A0 | 0% |
| Sample A1 | 0.1% |
| Sample A2 | 0.2% |
| Sample A3 | 0.3% |
| Sample A4 | 0.4% |
| Sample A5 | 0.5% |

Subsequently, the cell line contained in each of the samples A0 to A5 was cultured in a CO₂ incubator for 24 hours. The temperature in the CO₂ incubator was set at 37.0°C, and the concentration of CO₂ was set at 5.0%. Thereafter, 10 µL of a cell proliferation test reagent (manufactured by Seikagaku Corp.; product name: Tetra Color One) was added to each sample, and they were reacted in a CO₂ incubator for 2 hours. Thereafter, the absorbance at a wavelength of 450 nm (control wavelength: 600 nm) was measured using a microplate reader (manufactured by Bio-Rad Laboratories, Inc.), so as to measure the survival rate of the cell line. The survival rate was measured at time points of 24, 48, and 72 hours after the reaction with the cell proliferation test reagent.

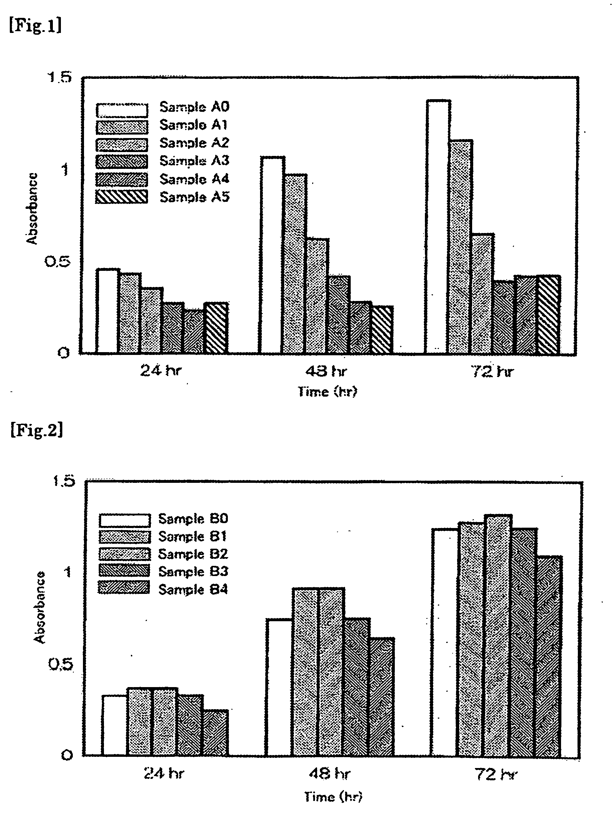

The measurement results are shown in Fig.1. As shown in Fig.1, it was confirmed that, as the concentration of DMSO used as a solvent was increased, radical scavenger action was expressed at a high level, and that the proliferation of cancer cells was suppressed. Thus, the DMSO concentration was set at 0.1% (sample A1), which had a minimum effect on the cancer cells. Accordingly, in the following experiments, the DMSO concentration was always set at 0.1%.

### [Determination of electroporation conditions]

Approximately 5,000 malignant melanoma cells (B16) and DMSO (0.1%) were added to each of multiple cuvettes (manufactured by BTX; product name: ECM399) having a pair of electrodes to result in a total amount of 600 µL. Thereafter, a DC pulse was applied to the electrodes of each cuvette so as to perform an electroporation operation. As for samples B0 to B4, the pulse voltage was set as shown in Table 2 below. For each sample, the number of pulse application was set at 10 times, the pulse width was set at approximately 10 msec, and the pulse duration was set at approximately 3 sec.

**[Table 2]**

| Sample name | Electric voltage |
|---|---|
| Sample B0 | 0 V |
| Sample B1 | 10 V |
| Sample B2 | 20 V |
| Sample B3 | 30 V |
| Sample B4 | 40 V |

Subsequently, the cell line contained in the samples B0 to B4 were cultured under the same conditions as those described in the "determination of solvent concentration" above, and the cell proliferation test reagent was reacted therewith. Then, the survival rate of the cells was measured at 24, 48, and 72 hours after the reaction.

The measurement results are shown in Fig.2. As shown in Fig.2, the result obtained from sample B3 was almost the same as those obtained from sample B0, on which electroporation had not been performed. Thus, the voltage applied during electroporation was set at 30 V, which had a minimum effect on the cancer cells. Therefore, the voltage applied during electroporation in the subsequent experiments was always set at 30V.

### [Examination of anti-cancer action 1 (without electroporation)]

Approximately 5,000 malignant melanoma cells (B16) were seeded into each well of a 96-well microplate, and DMSO (0.1%) and the rotaxane compound represented by formula (I) were then added thereto to result in a total amount of 100 µL. As for each of samples C0 to C2, the concentration of the rotaxane compound added was set as shown in Table 3.

**[Table 3]**

| Sample name | Concentration |
|---|---|
| Sample C0 | 0 nM |
| Sample C1 | 10 nM |
| Sample C2 | 100 nM |

Subsequently, the cell line contained in the samples C0 to C2 were cultured under the same conditions as those described in the "determination of solvent concentration" above, and the cell proliferation test reagent was reacted therewith. Then, the survival rate of the cells was measured at 0, 24, 48, and 72 hours after the reaction.

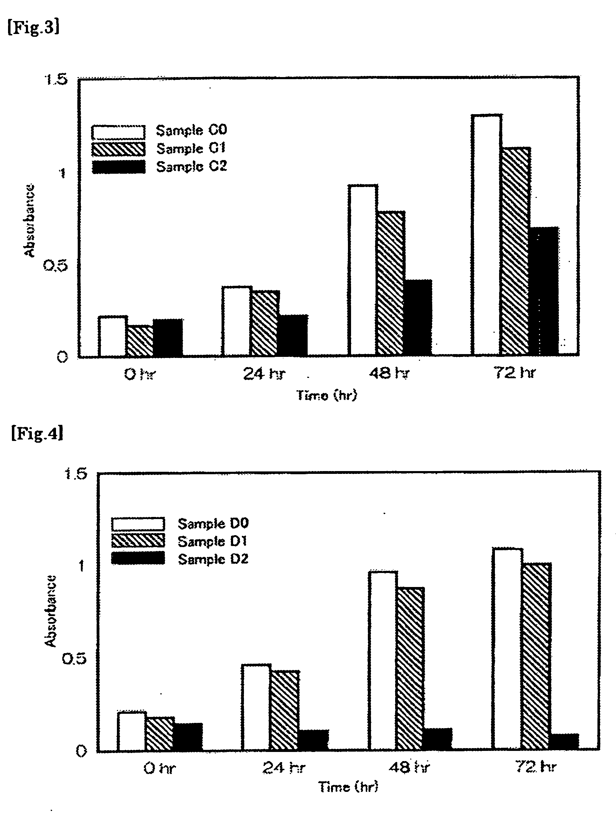

The measurement results are shown in Fig.3. As shown in Fig.3, it was confirmed that the higher the concentration of the rotaxane compound became, the smaller the number of cells after the culture became. From this result, it was confirmed that the rotaxane compound represented by formula (I) has an effect of retarding the proliferation of cancer cells. Though the details of the action mechanism of the rotaxane compound have not been clarified, it is considered that the rotaxane compound is associated with suppression of the cell proliferation, not time-dependently, but concentration-dependently. Thus, it is considered that the effect of the rotaxane compound does not depend on a cell cycle.

### [Examination of anti-cancer action 2 (with electroporation)]

Approximately 5,000 malignant melanoma cell line (B16), DMSO (0.1%), and the rotaxane compound represented by formula (I) were added to each of multiple cuvettes having a pair of electrodes, to result in a total amount of 600 µL. Thereafter, a DC pulse of 30 V was applied to the electrodes of each cuvette 10 times, so as to perform an electroporation operation. As for each of samples D0 to D2, the concentration of the rotaxane compound added was set as shown in Table 4 below.

**[Table 4]**

| Sample name | Concentration |
|---|---|
| Sample D0 | 0 nM |
| Sample D1 | 10 nM |
| Sample D2 | 100 nM |

Subsequently, the cell line contained in the samples D0 to D2 were cultured under the same conditions as those described in the "determination of solvent concentration" above, and the cell proliferation test reagent was reacted therewith. Then, the survival rate of the cells was measured at 0, 24, 48, and 72 hours after the reaction.

The measurement results are shown in Fig.4. As shown in Fig.4, the effect of the rotaxane compound to suppress the proliferation of cancer cells became significant, when electroporation was performed. In particular, in the case of the sample D2 in which the concentration of the rotaxane compound was set at 100 nM, it was confirmed that the cancer cells were reduced over time. From these results, it was confirmed that the proliferation of cancer cells was significantly suppressed by setting the concentration of the rotaxane compound at 100 nM or more and by performing electroporation.

Moreover, from the results regarding time and concentration, it was suggested that the present anti-cancer agent acts on cancer cells at a high concentration in a short amount of time.

### [Comparison with conventional anti-cancer agent]

The mRNA expression of an apoptosis-resistant gene Bcl-2, the mRNA expression of apoptosis-promoting genes Bax and Bad, and the mRNA expression of a cell death-executing gene Caspase-3 were analyzed based on ordinary β-actin proteins. The obtained results were compared with those of the conventional anti-cancer agent 5-FU. After extraction of total RNA, each mRNA expression was examined by a PCR (polymerase chain reaction) method. Each sample was electrophoresed by agarose gel electrophoresis, and fluorescence was then measured. Thereafter, the fluorescence ratio of each sample with a sample (a control), in which no anti-cancer agents were used, was obtained.

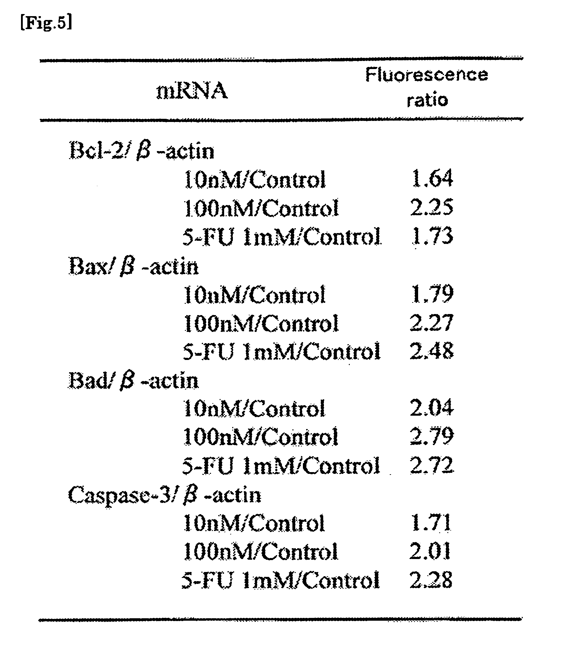

The results are shown in Fig.5. As shown in Fig.5, with regard to the obtained fluorescence ratio, the rotaxane compound was approximately similar to 5-FU. However, the applied amount of the rotaxane compound was one-tenth or less of that of 5-FU. Thus, it became clear that the anti-cancer action of the rotaxane compound is stronger than the conventional anti-cancer agent. From these results, it is considered that the rotaxane compound represented by formula (I) is useful as an anti-cancer agent.

### Industrial Applicability

The present invention is considered to be useful as an anti-cancer agent.

## Claims

1. A rotaxane compound represented by the following chemical formula (I) for use as an anti-cancer agent:

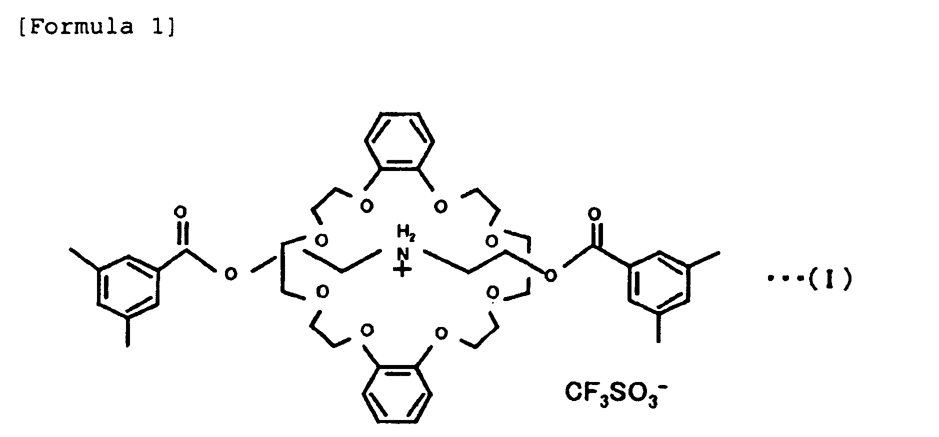

2. A compound according to claim 1 for use in treating cancer wherein the rotaxane compound is introduced into a cell by an electroporation method.

3. A composition for use as an anti-cancer agent which comprises a rotaxane compound according to claim 1 dissolved in a solution, and the concentration of said rotaxane compound in said solution is 10 nM or more.

4. A composition for use according to claim 3, wherein the concentration of said rotaxane compound in said solution is 100 nM or more.

5. A composition for use according to claim 3 or 4 wherein the solvent is DMSO.

6. A composition according to claim 3, 4 or 5 for use in treating cancer wherein said rotaxane compound is introduced into a cell by an electroporation method.

## Patentansprüche

1. Rotaxan-Verbindung, die durch die folgende chemische Formel (I) dargestellt wird, zur Verwendung als ein Antikrebsmittel:

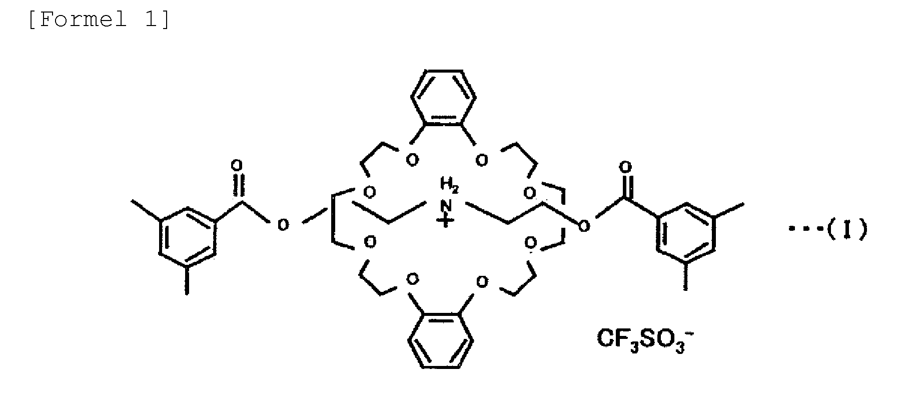

2. Verbindung gemäß Anspruch 1 zur Verwendung bei der Behandlung von Krebs, wobei die Rotaxan-Verbindung durch ein Elektroporationsverfahren in eine Zelle eingeführt wird.

3. Zusammensetzung zur Verwendung als Antikrebsmittel, welche eine Rotaxan-Verbindung gemäß Anspruch 1 gelöst in einer Lösung umfasst und wobei die Konzentration der Rotaxan-Verbindung in der Lösung 10 nM oder mehr ist.

4. Zusammensetzung zur Verwendung gemäß Anspruch 3, wobei die Konzentration der Rotaxan-Verbindung in der Lösung 100 nM oder mehr ist.

5. Zusammensetzung zur Verwendung gemäß Anspruch 3 oder 4, wobei das Lösungsmittel DMSO ist.

6. Zusammensetzung gemäß Anspruch 3, 4 oder 5 zur Verwendung bei der Behandlung von Krebs, wobei die Rotaxan-Verbindung durch ein Elektroporationsverfahren in eine Zelle eingeführt wird.

## Revendications

1. Composé de type rotaxane, représenté par la formule chimique (I) suivante, pour utilisation comme agent anti-cancéreux :

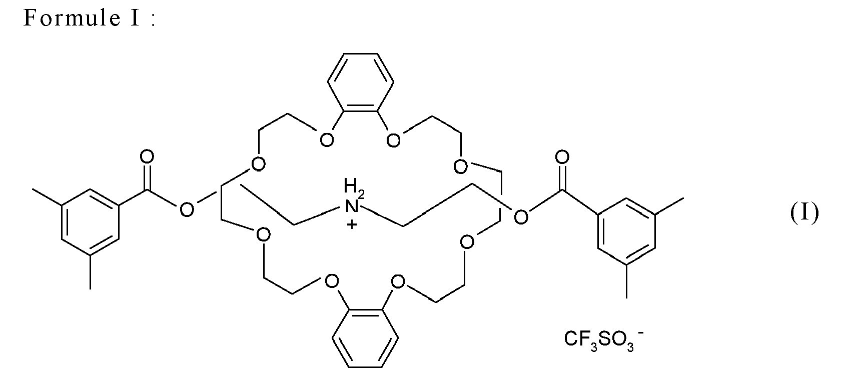

2. Composé conforme à la revendication 1, pour utilisation dans le traitement d'un cancer, lequel composé de type rotaxane est introduit dans une cellule par électroporation.

3. Composition pour utilisation comme agent anti-cancéreux, qui comprend un composé de type rotaxane, conforme à la revendication 1, à l'état dissous dans une solution, dans laquelle solution la concentration dudit composé de type rotaxane vaut 10 nM ou plus.

4. Composition pour utilisation, conforme à la revendication 3, dans laquelle la concentration dudit composé de type rotaxane dans ladite solution vaut 100 nM ou plus

5. Composition pour utilisation, conforme à la revendication 3 ou 4, dans laquelle le solvant est du diméthyl-sulfoxyde (DMSO).

6. Composition, conforme à la revendication 3, 4 ou 5, pour utilisation dans le traitement d'un cancer, ledit composé de type rotaxane étant introduit dans une cellule par électroporation.
